# EUROPEAN PATENT APPLICATION

(11) **EP 4 327 802 A1**
(43) Date of publication of application: **28.02.2024**
(21) Application number: 21937874.2
(22) Date of filing: 21.04.2021
(51) Int. Cl.: A61K 8/67, A61K 8/49, A61Q 19/08

(54) **COSMETIC**

(71) Applicant: Shiseido Company Ltd., Chuo-ku Tokyo 104-0061 (JP)
(72) Inventor: KOMAI Ryota, Tokyo 104-0061 (JP)
(74) Representative: Ter Meer Steinmeister & Partner
(86) International application number: PCT/JP2021/016197
(87) International publication number: WO 2022/224380

(57) **Abstract**

A cosmetic that achieves both a high wrinkle improvement effect and low irritation is provided.

A cosmetic according to the present invention includes: retinol; and an organic acid represented by general formula (I) below: 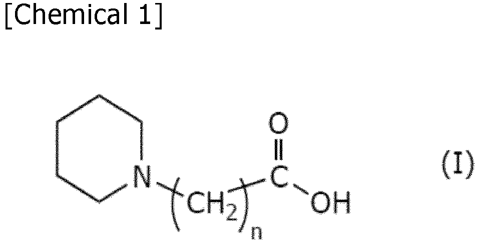 (in the formula (I), n represents an integer of 2 to 5)
or a salt thereof. The cosmetic may further include vitamin C as necessary.

## Description

### TECHNICAL FIELD

The present invention relates to a cosmetic capable of obtaining a sufficient wrinkle improvement effect while suppressing irritation.

### BACKGROUND ART

Antiaging is one of the major goals of skin care cosmetic products, and among them, wrinkles are the skin aging phenomenon that is of the greatest concern from the viewpoint of beauty. In recent years, retinol and the like have attracted attention as cosmetic drugs having a skin wrinkle improvement effect, and applications of retinol (vitamin A), which is a type of retinoid, retinoic acid, and the like to skin cosmetics have been studied. It is known that these compounds act on keratinocytes to exhibit a hyaluronic acid synthesis promoting effect, and exhibit a wrinkle improvement effect by an effect of increasing stratum corneum moisture. In particular, retinol is excellent as a cosmetic drug because retinol is milder in effect and does not exhibit strong side effects as compared with retinoic acid. However, it is known that retinol and derivatives thereof cause skin irritation when applied to the skin. Therefore, even when retinol or a derivative thereof is incorporated in a cosmetic, the formulation amount thereof cannot be increased, and it has been difficult to impart a sufficient wrinkle improvement effect.

### CITATION LIST

### PATENT LITERATURE

Patent Literature 1: Japanese Patent No. 4865251

### SUMMARY OF THE INVENTION

### OBJECT OF THE INVENTION

In order to solve the problems as described above, it has been desired to develop a technique capable of suppressing the skin irritation even when the formulation amount of retinol in cosmetics is increased.

### SOLUTION TO PROBLEM

According to the present invention, the following invention is provided.
[1] A cosmetic including:
   (A) retinol; and
   (B) an organic acid represented by general formula (I) below: 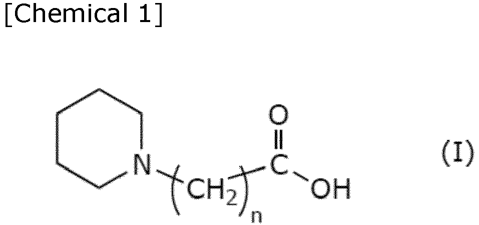
   (in the formula (I), n represents an integer of 2 to 5) or a salt thereof.
[2] The cosmetic according to [1], in which a formulation amount of the retinol is 0.01 to 0.5% by mass based on a total mass of the cosmetic.
[3] The cosmetic according to [1] or [2], in which a formulation amount of the organic acid or a salt thereof is 0.005 to 5% by mass based on a total mass of the cosmetic.
[4] The cosmetic according to any one of [1] to [3], in which a mass ratio of a formulation amount of the retinol to a formulation amount of the organic acid or a salt thereof is 0.005 to 1.
[5] The cosmetic according to any one of [1] to [4], in which the organic acid is 1-piperidinepropionic acid.
[6] The cosmetic according to any one of [1] to [5], further including vitamin C or a derivative thereof.
[7] The cosmetic according to [6], in which a formulation amount of the vitamin C or a derivative thereof is 0.005 to 1% by mass based on a total mass of the cosmetic.
[8] The cosmetic according to [6] or [7], in which a mass ratio of a formulation amount of the vitamin C or a derivative thereof to a formulation amount of the organic acid is 1/300 to 1.

### ADVANTAGEOUS EFFECTS OF THE INVENTION

According to the present invention, there is provided a cosmetic with a large formulation amount of retinol having suppressed skin irritation and a high wrinkle improvement effect.

### BEST MODE FOR CARRYING OUT THE INVENTION

Hereinafter, embodiments of the present invention will be described in detail.

The cosmetic according to the present invention contains (A) retinol and (B) a specific organic acid as essential components.

### [Retinol]

The cosmetic according to the present invention includes retinol. Retinol is used in the fields of cosmetic products, pharmaceuticals, quasi-drugs, and the like, and can be randomly selected from them also in the present invention.

There are several stereoisomers such as all-trans type, 13-cis type, and 9-cis type as retinol (also called vitamin A), and any of them can be used.

The formulation amount of retinol is not particularly limited as long as the formulation amount is an amount effective for exhibiting the effect of the component, but is usually preferably 0.01 to 0.5% by mass, and more preferably 0.02 to 0.1% by mass, based on the total mass of the cosmetic. By setting the content of retinol within such a range, the wrinkle improvement effect can be sufficiently obtained while suppressing irritation.

### [Organic acid or salt thereof]

The cosmetic according to the present invention includes a specific organic acid or a salt thereof. This organic acid is represented by the following general formula (I). 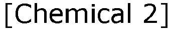 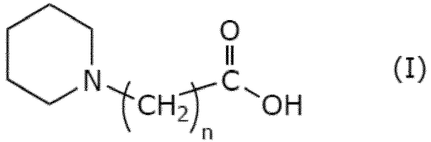 (in the formula (I), n represents an integer of 2 to 5)

Depending on the n number, the organic acid may be 1-piperidinepropionic acid (n = 2), 4-(1-piperidinyl)butanoic acid (n = 3), 5-(1-piperidinyl)pentanoic acid (n = 4), or 6-(1-piperidinyl)hexanoic acid (n = 5). Among these organic acids, 1-piperidinepropionic acid is particularly preferable because 1-piperidinepropionic acid not only suppresses the irritation caused by retinol but also improves the skin quality of the skin to make skin less likely to be rough and has the effect of suppressing wrinkle formation.

The salt of such an organic acid is not particularly limited, and examples of the inorganic salt include hydrochloride, sulfate, phosphate, hydrobromide, sodium salt, potassium salt, magnesium salt, calcium salt, and ammonium salt. Examples of the organic salt include acetate, lactate, maleate, fumarate, tartrate, citrate, methanesulfonate, p-toluenesulfonate, triethanolamine salt, diethanolamine salt, and amino acid salts.

The formulation amount of the organic acid or a salt thereof is not particularly limited, but is preferably 0.005 to 5% by mass, and more preferably 0.01 to 4% by mass, based on the total mass of the cosmetic. When the formulation amount of the organic acid or a salt thereof is within this range, it is possible to provide a cosmetic that can suppress irritation, has an excellent moisturizing feeling, has less sticky feeling, and has an excellent feeling of use.

In addition, in the present invention, in order to maintain an optimum balance between the wrinkle improvement effect by retinol and the irritation suppression effect by the organic acid or a salt thereof, the mass ratio (a/b) of the formulation amount (a) of retinol to the formulation amount (b) of the organic acid or a salt thereof is preferably 0.005 to 1, and more preferably 0.01 to 0.5.

### (Other components)

The cosmetic according to the present invention may include retinol and an organic acid or a salt thereof as essential components, but may include other components as necessary.

Among such components, vitamin C or a derivative thereof can be mentioned as a component which is preferably incorporated in the cosmetic product according to the present invention. Vitamin C is known as a nutrient and corresponds to L-ascorbic acid as a compound. Vitamin C or a derivative thereof is commonly used in cosmetic products. Vitamin C or a derivative thereof is known to exhibit a whitening effect and the like, and in the present invention, the effect of compensating for the irritation suppression effect of the above-described organic acid or a salt thereof is exhibited.

Vitamin C is water-soluble, and water-soluble, oil-soluble, and amphiphilic derivatives thereof are known, but water-soluble derivatives are preferably used. Specific examples thereof include 2-O-ethyl ascorbic acid, 3-O-ethyl ascorbic acid, sodium ascorbyl phosphate, magnesium ascorbyl phosphate, ascorbyl glucoside, glyceryl ascorbic acid, 3-glyceryl ascorbic acid, bisglyceryl ascorbic acid, and L-ascorbic acid palmitate.

The formulation amount of vitamin C or a derivative thereof is not particularly limited, but is preferably 0.005 to 1% by mass, and more preferably 0.008 to 0.2% by mass, based on the total mass of the cosmetic. When the formulation amount of vitamin C or a derivative thereof is within this range, it is possible to provide a cosmetic that is excellent in wrinkle improvement effect, has less irritation, and is excellent in feeling of use.

In the present invention, in order to optimally maintain the balance between the wrinkle improvement effect and the irritation suppression effect, the mass ratio (c/b) of the formulation amount (c) of vitamin C or a derivative thereof to the formulation amount (b) of the organic acid or a salt thereof is preferably 1/300 to 1, and more preferably 1/200 to 1/3.

In addition, in the cosmetic product according to the present invention, components usually used in cosmetic products, pharmaceuticals, and the like, for example, water, oils, surfactants, powders, coloring materials, alcohols, thickeners, chelating agents, silicones, antioxidants, ultraviolet absorbers, moisturizers, fragrances, various medicinal ingredients, preservatives, pH adjusting agents, neutralizing agents, and the like are appropriately incorporated as necessary.

In the cosmetic according to the present invention, a large amount of water in order to form a cosmetic liquid or an emulsion having a wrinkle improvement effect in particular is preferably incorporated. As the water, water used for cosmetic products, quasi-drugs, and the like can be used, and for example, purified water, ion-exchanged water, tap water, and the like can be used. The formulation amount of water varies depending on the formulation amount of other components, but is generally preferably 20 to 80% by mass, and more preferably 30 to 70% by mass, based on the total mass of the cosmetic.

The cosmetic according to the present invention can include a polyhydric alcohol. Such a polyhydric alcohol can be randomly selected as long as the effect of the present invention is not impaired. Specifically, the polyhydric alcohol is preferably one or two or more types selected from the group consisting of glycerin, diglycerin, polyglycerin, 1,3-butylene glycol, ethylene glycol, propylene glycol, dipropylene glycol, polyethylene glycol, polypropylene glycol, polyethylene glycol/polypropylene glycol, trimethylolethane, trimethylolpropane, erythritol, pentaerythritol, sorbitan, glucose, sorbitol, maltitol, sucrose, raffinose, hexylene glycol, 1,2-pentanediol, and trehalose.

The polyhydric alcohol exerts a moisturizing function and a warming sensation imparting function, and the formulation amount thereof is adjusted according to the purpose. In general, the formulation amount of the polyhydric alcohol is preferably 1 to 30% by mass, and more preferably 5 to 20% by mass, based on the total mass of the cosmetic.

In the present invention, the cosmetic according to the present invention can include a higher alcohol, and the higher alcohol is not particularly limited as long as the higher alcohol can be used in the fields of cosmetic products, pharmaceuticals, quasi-drugs, and the like. Specific examples thereof include lauryl alcohol, myristyl alcohol, cetyl alcohol, stearyl alcohol, and behenyl alcohol. In addition, mixtures thereof, for example, cetearyl alcohol (cetostearyl alcohol) which is a mixture of stearyl alcohol and cetyl alcohol, and the like are generally known, and can also be used.

In the case of incorporating a higher alcohol, one or two or more types described above can be used. In the present invention, it is preferable to use a mixture of two or more types of aliphatic alcohols, and it is more preferable to use such a combination that the melting point of the mixture is 60°C or higher. When the melting point is lower than 60°C, the temperature stability of the system may deteriorate depending on the formulation. In the present invention, for example, a combination of stearyl alcohol and behenyl alcohol is preferable.

The formulation amount of the higher alcohol is preferably 0.1 to 10% by mass, and more preferably 0.5 to 5% by mass with respect to the total mass of the cosmetic.

The cosmetic according to the present invention can include a surfactant. As the surfactant, various surfactants such as nonionic surfactants, cationic surfactants, anionic surfactants, amphoteric surfactants, and silicone surfactants are known, and any surfactant selected from them can be used. The formulation amount of the surfactant is preferably 0.1 to 20% by mass, and more preferably 0.3 to 5% by mass with respect to the total amount of the oil-in-water type emulsified cosmetic.

The cosmetic according to the present invention can include any oil depending on the purpose. The oil that can be used in the present invention is not particularly limited, and can be selected from those used in cosmetic products as long as stability is not impaired. Examples of preferable oils include polar oils such as hydrocarbon oils and ester oils, silicone oils, and liquid fats and oils.

Specifically, examples of the hydrocarbon oil include liquid paraffin, squalane, squalene, paraffin, isoparaffin, ceresin, isohexadecane, isododecane, and the like. Examples of polar oils such as ester oils include pentaerythrityl tetraethylhexanoate, cetyl ethylhexanoate, jojoba oil, di(phytosteryl/octyldodecyl)lauroyl glutamate, triisostearate, glyceryl diisostearate, triethylhexanoin, dimer dilinoleic acid (phytosteryl/behenyl), dimer dilinoleic acid (phytosteryl/isostearyl/cetyl/stearyl/isostearyl/cetyl/stearyl/behenyl), isopropyl palmitate, macadamia nut fatty acid phytosteryl, tetra (behenic acid/benzoic acid/ethylhexanoic acid)pentaerythrityl, ethylhexyl palmitate, myristyl myristate, isopropyl myristate, tripropylene glycol dipivalate, diisopropyl sebacate, and isodecyl neopentanoate. Examples of the silicone oil include chain silicones such as dimethylpolysiloxane, methylphenylpolysiloxane, and methylhydrogenpolysiloxane; cyclic silicones such as octamethylcyclotetrasiloxane, decamethylcyclopentasiloxane, and dodecamethylcyclohexasiloxane; silicone resins forming a three-dimensional network structure; and silicone rubbers. Examples of the liquid fats and oils include linseed oil, camellia oil, macadamia nut oil, corn oil, mink oil, olive oil, avocado oil, sasanqua oil, castor oil, safflower oil, rapeseed oil, soybean oil, peanut oil, triglycerin, glycerin trioctanoate, and glycerin triisopalmitate. When these oils are included, the oil-in-water type cosmetic is preferable.

The formulation amount of the oil is not particularly limited, but is preferably 5 to 40% by mass with respect to the total amount of the oil-in-water type emulsified cosmetic.

The cosmetic according to the present invention can be incorporated with further components depending on the purpose. Examples of such components include amino acids, bactericides, anti-inflammatory agents, astringents, animal and plant extracts, vitamins other than vitamin C and a derivative thereof, chelating agents, dyes, antioxidants, preservatives, and fragrances.

### [Cosmetic]

The cosmetic according to the present invention contains the essential components described above, but can be used in all dosage forms that can be taken by general cosmetics. For example, specifically, dosage forms such as a cosmetic liquid, an emulsion, a lotion, a cream, or an aerosol, can be employed. In any of the dosage forms, it is possible to provide a cosmetic having both less irritation and excellent feeling of use while achieving the wrinkle improvement effect.

### [Examples]

### [Examples 1 to 11 and Comparative Examples 1 to 4]

Cosmetics in each of Examples and Comparative Examples were prepared with the formulations shown in Table 1.

Furthermore, these cosmetics were evaluated for irritation and moisturizing feeling at the time of use according to the following criteria.

### [Evaluation method]

An actual use test by 20 expert panels was performed using the cosmetic of each example. The test items are lack of irritation and moisturizing feeling. Each expert panel evaluated each test item based on the following evaluation point criteria, and ranked by the sum of the evaluation points.

### Evaluation point criteria (irritation and moisturizing feeling):

5 points: Extremely excellent
4 points: Excellent
3 points: Ordinary
2 points: Poor
1 point: Extremely poor

### Evaluation rank of irritation

A+: 90 or more points in total
A: 80 or more points in total
B: Total score is 60 points or more and less than 80 points
C: Total score is 40 points or more and less than 60 points
D: Total point is less than 40 points

### Evaluation rank of moisturizing feeling

A: 80 or more points in total
B: Total score is 60 points or more and less than 80 points
C: Total score is 40 points or more and less than 60 points
D: Total point is less than 40 points

### [Table 1-1]

**Table 1-1**

| | | Example | | | | | | | |
|---|---|---|---|---|---|---|---|---|---|
| | | 1 | 2 | 3 | 4 | 5 | 6 | 7 | 8 |
| A | retinol | 0.02225 | 0.0555 | 0.0555 | 0.0555 | 0.083 | 0.083 | 0.0555 | 0.0555 |
| B | 1-piperidinepropionic acid | 3 | 0.1 | 1 | 3 | 1 | 0.1 | 1 | 1 |
| C | 2-O-ethyl ascorbic acid | | | | | | | 0.01 | 0.05 |
| | glycerin | 7 | 7 | 7 | 7 | 7 | 7 | 7 | 7 |
| | 1,3-butylene glycol | 7 | 7 | 7 | 7 | 7 | 7 | 7 | 7 |
| | carbomer | 0.12 | 0.12 | 0.12 | 0.12 | 0.12 | 0.12 | 0.12 | 0.12 |
| | potassium hydroxide | 0.02 | 0.02 | 0.02 | 0.02 | 0.02 | 0.02 | 0.02 | 0.02 |
| | beheneth-20 | 0.7 | 0.7 | 0.7 | 0.7 | 0.7 | 0.7 | 0.7 | 0.7 |
| | Cetearyl Alcohol | 0.9 | 0.9 | 0.9 | 0.9 | 0.9 | 0.9 | 0.9 | 0.9 |
| | behenyl alcohol | 3.3 | 3.3 | 3.3 | 3.3 | 3.3 | 3.3 | 3.3 | 3.3 |
| | Squalane | 11 | 11 | 11 | 11 | 11 | 11 | 11 | 11 |
| | Petrolatum | 2 | 2 | 2 | 2 | 2 | 2 | 2 | 2 |
| | pentaerythrityl tetraethylhexanoate | 15 | 15 | 15 | 15 | 15 | 15 | 15 | 15 |
| | dimeticone | 2 | 2 | 2 | 2 | 2 | 2 | 2 | 2 |
| | phenoxyethanol | q.s. | q.s. | q.s. | q.s. | q.s. | q.s. | q.s. | q.s. |
| | EDTA-3Na | q.s. | q.s. | q.s. | q.s. | q.s. | q.s. | q.s. | q.s. |
| | dibutylhydroxytoluene | q.s. | q.s. | q.s. | q.s. | q.s. | q.s. | q.s. | q.s. |
| | water | balance | balance | balance | balance | balance | balance | balance | balance |
| | mass ratio a/b | 0.00742 | 0.555 | 0.0555 | 0.0185 | 0.083 | 0.83 | 0.0555 | 0.0555 |
| | mass ratio cjb | 0 | 0 | 0 | 0 | 0 | 0 | 0.001 | 0.05 |
| eval. | irritation | A | B | A | A | A | B | A+ | A+ |
| | moisturizing feeling | A | B | A | A | A | B | A | A |

### [Table 1-2]

**Table 1-2**

| | | Example | | | Comparative Example | | | |
|---|---|---|---|---|---|---|---|---|
| | | 9 | 10 | 11 | 1 | 2 | 3 | 4 |
| A | retinol | 0.0555 | 0.0555 | 0.0555 | 0.02225 | | 0.02225 | |
| B | 1-piperidinepropionic acid | 1 | 0.1 | 3 | | 1 | | 1 |
| C | 2-O-ethyl ascorbic acid | 0.1 | 0.1 | 0.01 | | | 0.05 | 0.05 |
| | glycerin | 7 | 7 | 7 | 7 | 7 | 7 | 7 |
| | 1,3-butylene glycol | 7 | 7 | 7 | 7 | 7 | 7 | 7 |
| | carbomer | 0.12 | 0.12 | 0.12 | 0.12 | 0.12 | 0.12 | 0.12 |
| | potassium hydroxide | 0.02 | 0.02 | 0.02 | 0.02 | 0.02 | 0.02 | 0.02 |
| | beheneth-20 | 0.7 | 0.7 | 0.7 | 0.7 | 0.7 | 0.7 | 0.7 |
| | Cetearyl Alcohol | 0.9 | 0.9 | 0.9 | 0.9 | 0.9 | 0.9 | 0.9 |
| | behenyl alcohol | 3.3 | 3.3 | 3.3 | 3.3 | 3.3 | 3.3 | 3.3 |
| | Squalane | 11 | 11 | 11 | 11 | 11 | 11 | 11 |
| | Petrolatum | 2 | 2 | 2 | 2 | 2 | 2 | 2 |
| | pentaerythrityl tetraethylhexanoate | 15 | 15 | 15 | 15 | 15 | 15 | 15 |
| | dimeticone | 2 | 2 | 2 | 2 | 2 | 2 | 2 |
| | phenoxyethanol | q.s. | q.s. | q.s. | q.s. | q.s. | q.s. | q.s. |
| | EDTA-3Na | q.s. | q.s. | q.s. | q.s. | q.s. | q.s. | q.s. |
| | dibutylhydroxytoluene | q.s. | q.s. | q.s. | q.s. | q.s. | q.s. | q.s. |
| | water | balance | balance | balance | balance | balance | balance | balance |
| | mass ratio a/b | 0.0555 | 0.555 | 0.0185 | - | - | - | - |
| | mass ratio c/b | 0.1 | 1 | 0.0033 | - | - | - | - |
| eval. | irritation | A+ | A+ | A+ | D | A+ | D | A+ |
| | moisturizing feeling | A | B | A | D | A | D | A |

When Comparative Examples 1 and 3 are compared with Comparative Examples 2 or 4, it is found that irritation and moisturizing feeling tend to considerably deteriorate by incorporating retinol as conventionally reported. However, in Examples, even when the formulation amount of retinol is increased to about 2.5 times Comparative Example 1 or 3, improvement is achieved to a level comparable to the case where retinol is not incorporated. On the other hand, in Comparative Examples 2 and 4, since retinol is not included, it is less irritating, but it is clear that the wrinkle improvement effect, which is one of the purposes of the present invention, cannot be obtained. From the above results, it was found that, in the cosmetic according to the present invention, retinol can be incorporated at a high blending ratio, an excellent wrinkle improvement effect can be achieved, and at the same time, irritation is small, and an excellent moisturizing feeling can be obtained.

## Claims

1. A cosmetic comprising:
(A) retinol; and
(B) an organic acid represented by general formula (I) below: 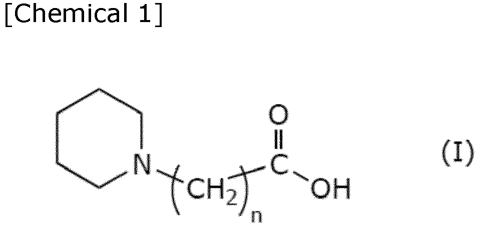 (in the formula (I), n represents an integer of 2 to 5) or a salt thereof.

2. The cosmetic according to claim 1, wherein a formulation amount of the retinol is 0.01 to 0.5% by mass based on a total mass of the cosmetic.

3. The cosmetic according to claim 1 or 2, wherein a formulation amount of the organic acid or a salt thereof is 0.005 to 5% by mass based on a total mass of the cosmetic.

4. The cosmetic according to any one of claims 1 to 3, wherein a mass ratio of a formulation amount of the retinol to a formulation amount of the organic acid or a salt thereof is 0.005 to 1.

5. The cosmetic according to any one of claims 1 to 4, wherein the organic acid is 1-piperidinepropionic acid.

6. The cosmetic according to any one of claims 1 to 5, further comprising vitamin C or a derivative thereof.

7. The cosmetic according to claim 6, wherein a formulation amount of the vitamin C or a derivative thereof is 0.005 to 1% by mass based on a total mass of the cosmetic.

8. The cosmetic according to claim 6 or 7, wherein a mass ratio of a formulation amount of the vitamin C or a derivative thereof to a formulation amount of the organic acid is 1/300 to 1.
